(19) Europäisches Patentamt
European Patent Office
Office européen des brevets

(11) Veröffentlichungsnummer: **0 103 218**
**B1**

(12) # EUROPÄISCHE PATENTSCHRIFT

(45) Veröffentlichungstag der Patentschrift: **30.04.86**

(51) Int. Cl.[4]: **C 07 D 471/04**, G 03 C 1/68 // (C07D471/04, 239:00, 221:00)

(21) Anmeldenummer: **83108311.8**

(22) Anmeldetag: **24.08.83**

(54) **10-Phenyl-1,3,9-triazaanthracene und diese enthaltendes photopolymerisierbares Gemisch.**

(30) Priorität: **02.09.82 DE 3232620**

(43) Veröffentlichungstag der Anmeldung: **21.03.84 Patentblatt 84/12**

(45) Bekanntmachung des Hinweises auf die Patenterteilung: **30.04.86 Patentblatt 86/18**

(84) Benannte Vertragsstaaten: **DE FR GB**

(56) Entgegenhaltungen:
**EP-A-0 005 750**
**CH-A-599 569**
**US-A-4 011 324**
**US-A-4 277 603**

**JOURNAL OF HETEROCYCLIC CHEMISTRY, Band 7, Februar 1970, Seiten 99-105, Albuquerque, US, D.E. O'BRIEN u.a.: "Synthesis of 10-deazariboflavin and related 2,4-dioxopyrimido(4,5-b)quinolines"**

(73) Patentinhaber: **HOECHST AKTIENGESELLSCHAFT, Postfach 80 03 20, D-6230 Frankfurt am Main 80 (DE)**

(72) Erfinder: **Bosse, Dieter, Dr., Berliner Strasse 12, D-6238 Hofheim/Ts. (DE)**
Erfinder: **Wingen, Rainer, Dr., Rauenthaler Weg 30, D-6000 Frankfurt 71 (DE)**
Erfinder: **Horn, Klaus, Dr., Lessingstrasse 53, D-6238 Hofheim/Ts. (DE)**
Erfinder: **Lutz, Walter, Dr., Eleonorenstrasse 130, D-6503 Mainz-Kastel (DE)**

EP 0 103 218 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue 10-Phenyl-1,3,9-triaza-anthracene sowie ein photopolymerisierbares Gemisch, das als wesentliche Bestandteile (a) ein polymeres Bindemittel, (b) eine polymerisierbare Verbindung mit mindestens zwei endständigen ethylenisch ungesättigten Gruppen und einem Siedepunkt oberhalb 100°C und (c) als Photoinitiator ein 10-Phenyl-1,3,9-triazaanthracen enthält.

Photopolymerisierbare Gemische aus den Bestandteilen (a) und (b) und einer mehrkernigen heterocyclischen Verbindung als Photoinitiator sind bekannt.

In der DE-C 20 27 467 (= GB-A 1 354 541) werden als Initiatoren bestimmte Derivate des Acridins und Phenazins beschrieben.

Aus der DE-C 20 39 861 (= US-A 3 765 898) sind ähnliche Gemische bekannt, die als Initiatoren Chinoxalin- oder Chinazolinderivate enthalten.

Alle diese Verbindungen zeigen hervorragende Initiatorwirkung unter Bestrahlung mit aktinischem Licht, insbesondere von Lichtquellen im nahen Ultraviolettbereich. Da in neuerer Zeit für Kopierzwecke wegen ihrer hohen Lichtausbeute verstärkt metallhalogeniddotierte Gasentladungslampen verwendet werden und diese gegenüber früher gebräuchlichen Lichtquellen, z. B. Quecksilberdampflampen, vermehrt Emissionen im Grenzbereich des sichtbaren Lichts um 400 nm und darüber aufweisen, sind die Absorptionen der bekannten hochwirksamen Initiatoren nicht mehr optimal auf die Emissionen dieser Lichtquellen abgestimmt. Auch ist die Variationsmöglichkeit durch Substitution der bekannten heterocyclischen Initiatoren begrenzt, d. h. es ist nach den gängigen Herstellungsverfahren nur in begrenztem Maße möglich, andere Eigenschaften, wie Löslichkeit im wäßrigen oder organischen Lösungsmitteln oder Verträglichkeit mit unterschiedlichen photopolymerisierbarem Gemischen, nach Wunsch durch gezielte Synthese zu verändern.

Aufgabe der Erfindung war es, neue Photoinitiatoren mit ähnlich hoher Wirksamkeit wie die bekannten vorzuschlagem, derem Lichtabsorption weiter in den kurzwelligen sichtbaren Bereich hineinreicht und in die sich im Laufe ihres Synthesewegs gezielt bestimmte Substituenten einführen lassen, die den Endprodukten unterschiedliche gewünschte Eigenschaften, wie Löslichkeit und Verträglichkeit mit anderen Komponenten, verleihen.

Gegenstand der Erfindung ist ein photopolymerisierbares Gemisch, das als wesentliche Bestandteile
(a) ein polymeres Bindemittel,
(b) eine polymerisierbare Verbindung mit mindestens zwei endständigen ethylenisch ungesättigten Gruppen und mit einem Siedepunkt oberhalb 100°C und
(c) als Photoinitiator eine mehrkernige N-heterocyclische Verbindung
enthält.

Das erfindungsgemäße Gemisch ist dadurch gekennzeichnet, daß die N-heterocyclische Verbindung der Formel A

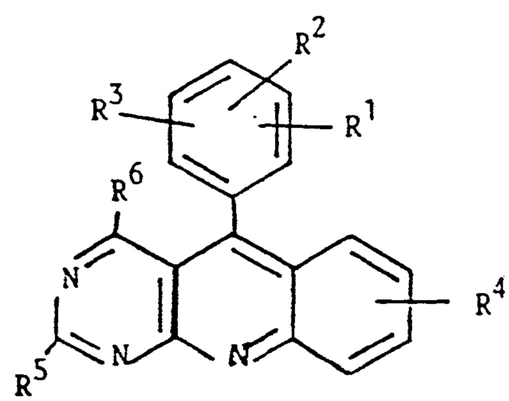

(A)

entspricht, worin

$R^1$, $R^2$ und $R^3$ gleich oder verschieden sind und jeweils ein Wasserstoff- oder Halogenatom, eine Alkyl- oder Alkoxygruppe mit jeweils 1 bis 6 Kohlenstoffatomen oder einen ankondensierten aromatischen Rest,

$R_4$ ein Wasserstoff- oder Halogenatom, eine Alkyl-, Alkoxy-, Halogenalkyl-, Alkylcarbonyl-, Alkoxycarbonylgruppe mit jeweils 1 bis 6 Kohlenstoffatomen in der Alkyl- oder Alkoxygruppe, eine Dialkylaminogruppe mit 2 bis 10 Kohlenstoffatomen oder einen ankondensierten aromatischen Rest und

$R^5$ und $R^6$ gleich oder verschieden sind und Hydroxy-, oder Alkoxygruppen mit 1 bis 6 Kohlenstoffatomen oder Dialkylaminogruppen mit 2 bis 10 Kohlenstoffatomen

bedeuten.

Erfindungsgemäß werden ferner neue 10-Phenyl-1,3,9-triazaanthracene der allgemeinen Formel A

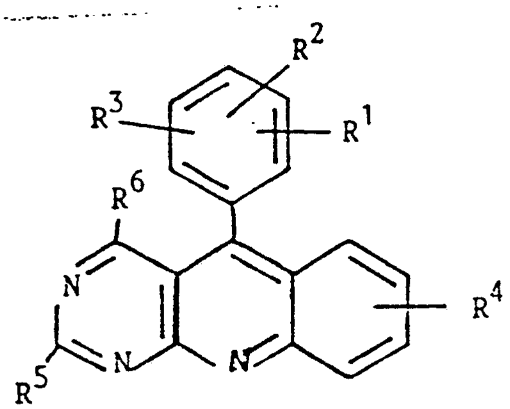

(A)

vorgeschlagen, worin

$R^1$ $R^2$ und $R^3$ gleich oder verschieden sind und jeweils ein Wasserstoff- oder Halogenatom, eine Alkyl- oder Alkoxygruppe mit jeweils 1 bis 6 Kohlenstoffatomen oder einen ankondensierten aromatischen Rest,

$R_4$ ein Wasserstoff- oder Halogenatom, eine Alkyl-, Alkoxy-, Halogenalkyl-, Alkylcarbonyl-, Alkoxycarbonylgruppe mit jeweils 1 bis 6 Kohlenstoffatomen in der Alkyl- oder Alkoxygruppe, eine Dialkylaminogruppe mit 2 bis 10 Kohlenstoffatomen oder einen ankondensierten aromatischen Rest und

$R^5$ und $R^6$ gleich oder verschieden sind und Hydroxy-, oder Alkoxygruppen mit 1 bis 6 Kohlenstoffatomen oder Dialkylaminogruppen mit 2 bis 10 Kohlenstoffatomen

bedeuten.

Die neuen Verbindungen können auf dem in der anliegenden Formelzeichnung skizzierten Weg hergestellt werden.

Sie absorbieren Licht im Spektralbereich von etwa 375 bis 430 nm und wirken bei Bestrahlung in diesem Spektralbereich als aktive Radikalstarter für die Photopolymerisation von Vinylverbindungen, auch in Gegenwart von Sauerstoff. Die neuen Photoinitiatoren haben die Eigenschaft, die thermische Polymerisation solcher Verbindungen in Abwesenheit von aktinischer Strahlung nicht zu initiieren. Sie sind somit ideal zur Herstellung lagerfähiger Kopierschichten geeignet.

Es ist hierbei von Vorteil, daß man weitgehend isomerenfreie Verbindungen (A) erhalten kann, indem man die Stufen (II) → (III) bzw. (III) → (IV) in der Weise führt, daß überwiegend nur eines der möglichen Substitutionsisomeren entsteht. Trennoperationen werden dadurch vermieden.

Die Reaktionsschritte stellen einfache, ungefährliche und meist mit hoher Ausbeute ablaufende Reaktionen dar, die als Endprodukte in der Literatur noch nicht beschriebene Verbindungen liefern.

Die Alkyl- und Alkoxygruppen, die als Substituenten in Betracht kommen, haben vorzugsweise 1 bis 3 Kohlenstoffatome; Methyl- und Methoxygruppen werden besonders bevorzugt.

Die Dialkylaminogruppen haben vorzugsweise 2 bis 6 Kohlenstoffatome. Als Halogenatome werden Fluor- und Chloratome, im Falle von $R^4$ besonders Fluoratome, bevorzugt. Als Halogenalkylgruppen werden solche mit 1 bis 3 Kohlenstoffatomen, insbesondere Methylgruppen, bevorzugt, die vorzugsweise vollständig halogeniert sind. Als Halogenatome kommen die gleichen in Betracht, wie sie oben genannt sind. In den Alkyl- und Alkoxycarbonylgruppen können die gleichen Alkyl- und Alkoxygruppen vorliegen wie oben angegeben. Als ankondensierte aromatische Reste werden Benzoreste bei weitem bevorzugt.

Von den Verbindungen der Formel A werden diejenigen besonders bevorzugt, in denen $R^6$ eine OH- oder Alkoxygruppe ist. $R^1$, $R^2$ und $R^3$ sind bevorzugt Wasserstoff oder Halogenatome, Alkyl- oder Alkoxygruppen oder ankondensierte Benzogruppen. Wenn einer der Reste eine Benzogruppe ist, sind die beiden anderen bevorzugt Wasserstoffatome. Besonders bevorzugt werden Verbindungen, in denen alle drei Reste $R^1$ bis $R^3$ Wasserstoffatome sind.

Die Photoinitiatoren werden im allgemeinen in einer Menge von 0,01 bis 10, bevorzugt von 0,1 bis 5 Gew.-%, bezogen auf die Bestandteile der photopolymerisierbaren Schicht, zugesetzt.

Für die Zwecke der Erfindung geeignete photopolymerisierbare Monomere sind bekannt und z.B. in den USA-Patentschriften 2 760 863 und 3 060 023 beschrieben.

Bevorzugte Beispiele sind Acryl- und Methacrylsäureester, wie Diglycerindiacrylat, Polyethylenglykoldimethacrylat, Acrylate und Methacrylate von Trimethylolethan, Trimethylolpropan und Pentaerythrit und von mehrwertigen alicyclischen Alkoholen. Mit Vorteil werden auch Umsetzungsprodukte von Diisocyanaten mit Partialestern mehrwertiger Alkohole eingesetzt. Derartige Monomere sind in den DE-A 28 64 079, 23 61 041 und 28 22 190 beschrieben.

Der Mengenanteil der Schicht an Monomeren beträgt im allgemeinen etwa 10 bis 80, vorzugsweise 20 bis 60 Gew.-%.

Als Bindemittel können eine Vielzahl löslicher organischer Polymerisate Einsatz finden. Als Beispiele seien genannt: Polyamide, Polyvinylester, Polyvinylacetale, Polyvinylether, Epoxidharze, Polyacrylsäureester, Polymethacrylsäureester, Polyester, Alkydharze, Polyacrylamid, Polyvinylalkohol, Polyethylenoxid, Polydimethylacrylamid, Polyvinylpyrrolidon, Polyvinylmethylformamid, Polyvinylmethylacetamid sowie

Mischpolymerisate der Monomeren, die die aufgezählten Homopolymerisate bilden.

Ferner kommen als Bindemittel Naturstoffe oder umgewandelte Naturstoffe in Betracht, z. B. Gelatine und Celluloseether.

Mit besonderem Vorteil werden Bindemittel verwendet, die wasserunlöslich, aber in wäßrig-alkalischen Lösungen löslich oder mindestens quellbar sind, da sich Schichten mit solchen Bindemitteln mit den bevorzugten wäßrigalkalischen Entwicklern entwickeln lassen. Derartige Bindemittel können z. B. die folgenden Gruppen enthalten: -COOH, $-PO_3H_2$, $-SO_3H$; $-SO_2NH-$ und $-SO_2-NH-CO-$.

Als Beispiele hierfür seien genannt: Maleinatharze, Polymerisate aus N-(p-Tolyl-sulfonyl)-carbaminsäure-(β-methacryloyloxy-ethyl)ester und Mischpolymerisate dieser und ähnlicher Monomerer mit anderen Monomeren sowie Styrol-Maleinsäureanhydrid-Mischpolymerisate. Alkylmethacrylat-Methacrylsäure-Mischpolymerisate und Mischpolymerisate aus Methacrylsäure, Alkylmethacrylaten und Methylmethacrylat und/oder Styrol, Acrylnitril u. a., wie sie in den DE-A 20 64 080 und 23 63 806 beschrieben sind, werden bevorzugt.

Die Menge des Bindemittels beträgt im allgemeinen 28 bis 90, vorzugsweise 40 bis 80 Gew.-% der Bestandteile der Schicht.

Die photopolymerisierbaren Gemische können je nach geplanter Anwendung und je nach den gewünschten Eigenschaften verschiedenartige Stoffe als Zusätze enthalten. Beispiele sind:

Inhibitoren zur Verhinderung der thermischen Polymerisation der Monomeren,
Wasserstoffdonatoren, die sensitometrischen Eigenschaften derartiger Schichten modifizierende Stoffe,
Farbstoffe, gefärbte und ungefärbte Pigmente,
Farbbildner,
Indikatoren,
Weichmacher usw.

Diese Bestandteile sind zweckmäßig so auszuwählen, daß sie in dem für den Initiierungsvorgang wichtigen aktinischen Strahlungsbereich möglichst wenig absorbieren.

Als aktinische Strahlung soll im Rahmen dieser Beschreibung jede Strahlung verstanden werden, deren Energie mindestens der des kurzwelligen sichtbaren Lichts entspricht. Geeignet ist langwellige UV-Strahlung, aber auch Elektronen-, Röntgen- und Laserstrahlung.

Das photopolymerisierbare Gemisch kann für die verschiedensten Anwendungen Einsatz finden, beispielsweise zur Herstellung von Sicherheitsglas, von Lacken, die durch Licht oder Korpuskularstrahlen, z. B. Elektronenstrahlen, gehärtet werden, auf dem Dentalgebiet und insbesondere als lichtempfindliches Kopiermaterial auf dem Reproduktionsgebiet.

Die detaillierte Beschreibung der Erfindung beschränkt sich auf dieses Anwendungsgebiet, jedoch ist die Erfindung nicht hierauf beschränkt. Als Anwendungsmöglichkeiten auf diesem Gebiet seien genannt: Kopierschichten für die photomechanische Herstellung von Druckformen für den Hochdruck, den Flachdruck, den Tiefdruck, den Siebdruck, von Reliefkopien, z. B. Herstellung von Texten in Blindenschrift, von Einzelkopien, Gerbbildern, Pigmentbildern usw.. Weiter sind die Gemische zur photomechanischen Herstellung von Ätzreservagen, z. B. für die Fertigung von Namensschildern, von kopierten Schaltungen und für das Formteilätzen, anwendbar. Besondere Bedeutung haben die erfindungsgemäßen Gemische als Kopierschichten für die photomechanische Herstellung von Flachdruckformen und von Ätzreservagen, insbesondere als vorsensibilisierte Materialien.

Die gewerbliche Verwertung des Gemischs für die genannten Anwendungszwecke kann in der Form einer flüssigen Lösung oder Dispersion, z. B. als Photoresistlösung, erfolgen, die vom Verbraucher selbst auf einen individuellen Träger, z. B. zum Formteilätzen, für die Herstellung kopierter Schaltungen, von Siebdruckschablonen und dgl., aufgebracht wird. Das Gemisch kann auch als feste lichtempfindliche Schicht auf einem geeigneten Träger in Form eines lagerfähig vorbeschichteten lichtempfindlichen Kopiermaterials, z. B. für die Herstellung von Druckformen, vorliegen, Ebenso ist es für die Herstellung von Trockenresist geeignet.

Es ist im allgemeinen günstig, die Gemische während der Lichtpolymerisation dem Einfluß des Luftsauerstoffes weitgehend zu entziehen. Im Fall der Anwendung des Gemischs in Form dünner Kopierschichten ist es empfehlenswert, einen geeigneten, für Sauerstoff wenig durchlässigen Deckfilm aufzubringen. Dieser kann selbsttragend sein und vor der Entwicklung der Kopierschicht abgezogen werden. Für diesen Zweck sind z. B. Polyesterfilme geeignet. Der Deckfilm kann auch aus einem Material bestehen, das sich in der Entwicklerflüssigkeit löst oder mindestens an den nicht gehärteten Stellen bei der Entwicklung entfernen läßt. Hierfür geeignete Materialien sind z. B. Wachse, Polyvinylalkohol, Polyphosphate, Zucker usw..

Als Schichtträger für mit dem erfindungsgemäßen Gemisch hergestellte Kopiermaterialien sind beispielsweise Aluminium, Stahl, Zink, Kupfer und Kunststoff-Folien, z. B. aus Polyethylenterephthalat oder Cellulosacetat, sowie Siebdruckträger, wie Perlongaze, geeignet.

Die Herstellung der lichtempfindlichen Materialien unter Verwendung des erfindungsgemäßen Gemischs erfolgt in bekannter Weise.

So kann man dieses in einem Lösungsmittel aufnehmen und die Lösung bzw. Dispersion durch Gießen, Sprühen, Tauchen, Antrag mit Walzen usw. auf den vorgesehenen Träger als Film antragen und anschließend antrocknen. Dicke Schichten (z. B. von 250µm und darüber) werden vorteilhaft durch Extrudieren oder Verpressen als selbsttragende Folie hergestellt, welche dann ggf. auf den Träger laminiert wird. Im Falle von Trockenresist werden Lösungen des Gemischs auf transparente Träger aufgebracht und angetrocknet. Die lichtempfindlichen Schichten - Dicke etwa zwischen 10 und 100µm - werden dann gleichfalls zunächst durch Laminieren zusammen mit dem temporären Träger auf das gewünschte Substrat auflaminiert.

Die Verarbeitung der Kopiermaterialien wird in bekannter Weise vorgenommen. Zur Entwicklung werden sie mit einer geeigneten Entwicklerlösung, bevorzugt einer schwach alkalischen wäßrigen Lösung, behandelt, wobei die unbelichteten Anteile der Schicht entfernt werden und die belichteten Bereiche der Kopierschicht auf dem Träger zurückbleiben.

Im folgenden werden Beispiele für das erfindungsgemäße Gemisch angegeben. Dabei wird zunächst die Herstellung einer Anzahl neuer Photoinitiatoren gemäß der Erfindung beschrieben. Da die Endstufe der Cyclisierung zur Verbindung (A) von drei verschiedenen Zwischenprodukten (IV), (V) und (VI) ausgehen kann, ist eine große Vielfalt von Variationen möglich.

In den Herstellungsvorschriften und den nachfolgenden Beispielen stehen Gewichtsteile (Gt) und Volumenteile (Vt) im Verhältnis von g zu ccm. Sofern nicht anders angegeben, verstehen sich Prozent- und Mengenverhältnisse in Gewichtseinheiten.

Arbeitsvorschrift zur Herstellung der Verbindung (II) (2,4,6-Trichlor-pyrimidin-5-carbonsäurechlorid)

Zur Lösung von 2 mol 2,4,6-Trichlor-pyrimidin-5-carb-aldehyd in 2 1 Tetrachlormethan werden bei 77°C 0,2 g Azo-bisisobutyronitril (AIBN) und 2 mol Sulfurylchlorid gegeben, und die Mischung wird 15 Stunden auf Siedetemperatur gehalten, wobei jeweils nach 3 Stunden 16 ml Sulfurylchlorid und 0,1 g AIBN zugefügt werden. Nach Abkühlen und Filtrieren wird das Lösungsmittel im Vakuum entfernt und der Rückstand destilliert. Es resultieren 391,4 g farbloses Destillat (71-73° C/0,2 mbar) vom Schmp. 44° C entsprechend einer Ausbeute von 79,6 % d.Th. an Verbindung (II).

Allgemeine Vorschrift zur Herstellung der Verbindungen der Formel (III)

1 mol Acylchlorid (II) wird mit 5-7 mol der entsprechenden aromatischen Verbindung $C_6H_5R^1R^2R^3$ und 1,2 mol Aluminiumchlorid in überschüssiger aromatischer Verbindung als Lösungsmittel oder den für Friedel-Crafts-Acylierungen bekannten Lösungsmitteln zur Reaktion gebracht, das Reaktionsgemisch mit HCl/Eis hydrolysiert, die wäßrige Phase erschöpfend mit einem geeigneten Lösungsmittel extrahiert und der nach Abdestillieren des Lösungsmittels erhaltene Extraktionsrückstand umkristallisiert.

**Tabelle 1**

| Verbindung Nr. | $R^1$ | $R^2$ | $R^3$ | Schmp. °C | Ausbeute % |
|---|---|---|---|---|---|
| III a | H | H | H | 148 | 78,5 |
| b | 4-Cl | H | H | 173 | 81,2 |
| c | 4-$CH_3$ | H | H | 138 | 59,1 |
| d | 2-$CH_3$ | 4-$CH_3$ | H | 128 | 73,3 |
| e | 2-$CH_3$ | 5-$CH_3$ | H | 165 | 83,7 |
| f | 2-$CH_3$ | 4-$CH_3$ | 6-$CH_3$ | 164 | 88,3 |
| g | 4-$OCH_3$ | H | H | 152 | 42,0 |
| h | 2,3-Benzo | | H | 197 | 62,9 |

Allgemeine Vorschrift zur Herstellung der Verbindungen der allgemeinen Formel (IV)

1 mol (III) wird in einem unter den Reaktionsbedingungen inerten Lösungsmittel, vorzugsweise Tetrahydrofuran, Dioxan oder Dialkylether, bei 0 bis 40° C, vorzugsweise 20° C, mit 1 mol aromatischem Amin und 1,1 mol eines tertiären Amins, vorzugsweise Triethylamin, umgesetzt. Das Triethylaminhydrochlorid wird abfiltriert und der Rückstand nach Abdestillation des Lösungsmittels durch Verrühren mit einem geeigneten Lösungsmittel zur Kristallisation gebracht.

**Tabelle 2**
$R^2 = R^3 = H$

| Verbindung Nr. | $R^1$ | $R^4$ | Schmp. °C | Ausbeute % |
|---|---|---|---|---|
| IV a | H | H | 128 | 76,4 |
| b | H | 2-$OCH_3$ | 132 | 67,4 |
| c | H | 4-$OCH_3$ | 86 | 40,1 |
| d | H | 4-F | 163 | 69,0 |
| e | H | 4-$CO_2CH_3$ | 182 | 42,7 |
| f | H | 4-$CF_3$ | 183 | 33,5 |
| g | 4-Cl | 4-Cl | 177 | 81,3 |
| h | 4-Cl | 2-$OCH_3$ | 162 | 59,2 |
| i | H | 4-$N(C_2H_5)_2$ | 148 | 55,4 |
| j | H | 4-$COCH_3$ | 150 | 44,0 |
| k | H | 3,4-Benzo | 142 | 73,6 |

Allgemeine Vorschrift zur Herstellung der Verbindungen der allgemeinen Formel (V)

0,1 mol (IV) wird entweder mit 0,1 mol Alkalialkoholat $MeR^5$ im entsprechenden Alkohol 5 Stunden bei Raumtemperatur umgesetzt, der Feststoff abgesaugt, mit Alkohol und Wasser gewaschen und getrocknet oder mit 0,3 mol Amin $R^5H$ in Tetrahydrofuran bei Raumtemperatur 1-5 Stunden gerührt, vom Aminhydrochlorid abfiltriert und das Filtrat nach Entfernung des Lösungsmittels durch Verrühren mit einem geeigneten Lösungsmittel zur Kristallisation gebracht.

**Tabelle 3**
$R^2 = R^3 = H$

| Verbindung Nr. | $R^1$ | $R^4$ | $R^5$ | $R^6$ | Schmp. °C | Ausbeute % |
|---|---|---|---|---|---|---|
| V a | H | H | $OCH_3$ | Cl | 122 | 78,2 |
| b | H | H | $N(C_2H_5)_2$ | Cl | 137 | 49,9 |
| c | H | 4-F | $N(C_2H_5)_2$ | Cl | 104 | 85,3 |
| d | H | 4-$CF_3$ | $N(C_2H_5)_2$ | Cl | 131 | 80,7 |
| e | H | 4-$CO_2CH_3$ | $N(C_2H_5)_2$ | Cl | 131 | 86,5 |
| f | H | 4-$COCH_3$ | $N(C_2H_5)_2$ | Cl | 144 | 66,6 |
| g | H | 4-$OCH_3$ | $N(C_2H_5)_2$ | Cl | 88 | 86,3 |

Allgemeine Vorschrift zur Herstellung der Verbindungen der allgemeinen Formel (VI)

Zur Einführung von Substituenten in 2- und/oder 4-Stellung des Pyrimidinrings wird 0,1 mol (IV) bzw. (V) entweder mit 0,4 mol Alkalialkoholat im entsprechenden Alkohol 10 bis 15 Stunden auf 70°C erhitzt, der Feststoff nach dem Erkalten abgesaugt, mit Alkohol und Wasser gewaschen und umkristallisiert oder 0,1 mol (IV) bzw. (V) mit 0,5 mol Amin in Tetrahydrofuran oder Acetonitril 12 bis 16 Stunden auf Siedetemperatur erhitzt, das Aminhydrochlorid durch Filtration abgetrennt und das Filtrat nach Entfernung des Lösungsmittels durch Verrühren mit einem geeigneten Lösungsmittel zur Kristallisation gebracht.

**Tabelle 4**

$R^2 = R^3 = H$

| Verbindung Nr. | $R^1$ | $R^4$ | $R^5$ | $R^6$ | Schmp. °C | Ausbeute % |
|---|---|---|---|---|---|---|
| VI a | H | H | $OCH_3$ | $OCH_3$ | 140 | 87,2 |
| b | H | H | $N(C_2H_5)_2$ | $OCH_3$ | 106 | 86,3 |
| c | H | 2-$OCH_3$ | $OCH_3$ | $OCH_3$ | 183 | 84,3 |
| d | H | 4-F | $OCH_3$ | $OCH_3$ | 146 | 92,0 |
| e | H | 4-$CO_2CH_3$ | $N(C_2H_5)_2$ | $OCH_3$ | 142 | 58,2 |
| f | H | 4-$CO_2CH_3$ | $OCH_3$ | $OCH_3$ | 138 | 70,4 |
| g | H | 4-$CF_3$ | $N(C_2H_5)_2$ | $OCH_3$ | 124 | 81,7 |
| h | H | 4-$CF_3$ | $OCH_3$ | $OCH_3$ | 142 | 76,5 |
| i | 4-Cl | 4-Cl | $OCH_3$ | $OCH_3$ | 181 | 88,6 |
| j | 4-Cl | 2-$OCH_3$ | $OCH_3$ | $OCH_3$ | 135 | 89,9 |
| k | H | 4-$OCH_3$ | $OCH_3$ | $OCH_3$ | 137 | 93,3 |
| l | H | 4-$OCH_3$ | $N(C_2H_5)_2$ | $OCH_3$ | 95 | 75,8 |
| m | H | 4-F | $N(C_2H_5)_2$ | $OCH_3$ | 86 | 91,8 |
| n | H | 4-$N(C_2H_5)_2$ | $OCH_3$ | $OCH_3$ | 109 | 88,2 |
| o | H | 4-$CO_2CH_3$ | $N(C_2H_5)_2$ | $N(C_2H_5)_2$ | 124 | 74,0 |
| p | H | 4-$COCH_3$ | $N(C_2H_5)_2$ | $OCH_3$ | 148 | 85,8 |
| q | H | 4-$COCH_3$ | $OCH_3$ | $OCH_3$ | 138 | 74,3 |
| r | H | 3,4-Benzo | $OCH_3$ | $OCH_3$ | 123 | 83,6 |

Allgemeine Vorschrift zur Herstellung der Verbindungen der allgemeinen Formel (A)

1 Gt (IV) bzw. (V) bzw. (VI) wird bei 40 - 50°C in eine Mischung von 10 Vt Trifluoressigsäure und 1 Vt Schwefelsäure (98 %) gegeben und bis zum Ende der Reaktion bei gleicher Temperatur gerührt, auf 50 Vt Eis gegeben, mit Alkali auf pH 8 gestellt, der Feststoff abgetrennt, neutral gewaschen und aus Dimethylformamid umkristallisiert. Bei der Umsetzung erfolgt die Cyclisierung zum Pyridinring. Wenn von (IV) oder (V) ausgegangen wird, werden zugleich die Chloratome $R^5$ und/oder $R^6$ durch OH-Gruppen ersetzt.

**Tabelle 5**
$R_2 = R_3 = H$

| Verbindung Nr. | $R^1$ | $R^4$ | $R^5$ | $R^6$ | Schmp. °C | Ausbeute % |
|---|---|---|---|---|---|---|
| A a | H | H | OH | OH | 350 | 81,9 |
| b | H | H | $OCH_3$ | $OCH_3$ | 207 | 55,1 |
| c | H | H | $OCH_3$ | OH | 250 | 85,2 |
| d | H | H | $N(C_2H_5)_2$ | OH | 329 | 66,4 |
| e | H | H | $N(C_2H_5)_2$ | $OCH_3$ | 199 | 50,4 |
| f | H | 9-$OCH_3$ | $OCH_3$ | $OCH_3$ | 208 | 72,6 |
| g | H | 7-$OCH_3$ | $N(C_2H_5)_2$ | OH | 288 | 48,4 |
| h | H | 7-F | $N(C_2H_5)_2$ | OH | 330 | 35,0 |

FIG6/180

**Fortsetzung Tabelle 5**
$R_2 = R_3 = H$

| Verbindung Nr. | $R^1$ | $R^4$ | $R^5$ | $R^6$ | Schmp. °C | Ausbeute % |
|---|---|---|---|---|---|---|
| A i | H | 7-F | $OCH_3$ | $OCH_3$ | 210 | 15,8 |
| j | H | 7-$CO_2CH_3$ | $N(C_2H_5)_2$ | OH | 259 | 56,7 |
| k | H | 7-$CO_2CH_3$ | $N(C_2H_5)_2$ | $OCH_3$ | 224 | 33,3 |
| l | H | 7-$CO_2CH_3$ | $OCH_3$ | $OCH_3$ | 268 | 47,2 |
| m | H | 7-$CF_3$ | $N(C_2H_5)_2$ | OH | 237 | 14,3 |
| n | H | 7-$CF_3$ | $N(C_2H_5)_2$ | $OCH_3$ | 199 | 5,7 |
| o | H | 7-$CF_3$ | $OCH_3$ | $OCH_3$ | 221 | 37,5 |
| p | 4-Cl | 7-Cl | OH | OH | 350 | 75,2 |
| q | 4-Cl | 7-Cl | $OCH_3$ | $OCH_3$ | 244 | 41,9 |
| r | 4-Cl | 9-$OCH_3$ | $OCH_3$ | $OCH_3$ | 248 | 73,3 |

Die Starteraktivität aller Initiatoren gemäß Formel A a-r ist in einer Reihe von Beispielen tabellarisch zusammengefaßt.

**Beispel 1**

Eine Lösung von

4,0 Gt eines Methylmethacrylat/Methacrylsäure-Copolymerisats (Säurezahl ca. 110),

4,0 Gt Trimethylolethantriacrylat,

0,08 Gt eines blauen Azofarbstoffs, erhalten durch Kuppeln von 2,4-Dinitro-6-chlorbenzoldiazoniumsalz mit 2-Methoxy-5-acetylamino-N-cyanoethyl-N-hydroxyethyl-anilin, und

0,21 Gt Initiator in

38 Gt Ethylenglykolmonoethylether und

18 Gt Butylacetat

wird auf elektrolytisch aufgerauhtes und anodisch oxydiertes 0,3 mm starkes Aluminium so aufgeschleudert und getrocknet, daß ein Schichtgewicht von 25 $g/m^2$ erhalten wird.

Nach dem Trocknen wird die Photopolymerschicht mit einer Lösung von

5 Gt Polyvinylalkohol (12 % Restacetylgruppen; K-Wert 8) in

95 Gt entsalztem Wasser

überschichtet und getrocknet, so daß eine abziehbare Deckschicht von ca. 5 $g/m^2$ entsteht.

Anschließend wird mit einer 5 kW-Metallhalogenidlampe 40 Sekunden unter einem 13-stufigen Belichtungskeil belichtet. Nach dem Belichten wird die Platte kurzzeitig auf 90°C erwärmt.

Die belichtete Photopolymerschicht wird ca. 1 Minute mittels eines Plüschtampons mit einem Entwickler aus

1,5 Gt Natriummetasilikat x 9 $H_2O$,

0,01 Gt Polyoxyethylenether von Kokosfettalkohol mit etwa 8 Oxyethyleneinheiten und

98,3 Gt entsalztem Wasser

entwickelt.
Nach Wasserspülung wird mit 1 %iger Phosphorsäure sauer gestellt und mit Fettfarbe eingefärbt.
Nach dem Einfärben wird mit handelsüblicher Konservierungslösung gummiert und getrocknet. Auf einer Offsetdruckmaschine werden Auflagen um 100.000 erhalten.
Es werden folgende Lichtempfindlichkeiten erhalten:

| Verbindung | entwickelte Vollstufen |
|---|---|
| A a | 2 |
| b | 1 |
| c | 1 |
| d | 3 |
| e | 2 |
| f | 2 |
| g | 4 |
| h | 2 |
| i | 5 |

| Verbindung | entwickelte Vollstufen |
|---|---|
| j | 3 |
| k | 3 |
| l | 5 |
| m | 4 |
| n | 3 |
| o | 5 |
| p | 2 |
| q | 1 |
| r | 2 |

**Beispiel 2**
4 Lösungen von jeweils
5,6 Gt des Umsetzungsproduktes aus 1 mol 2,2,4-Trimethyl-hexamethylendiisocyanat und 2 mol 2-Hydroxyethylmethacrylat,
6,5 Gt eines Terpolymerisats aus Styrol, n-Hexylmethacrylat und Methacrylsäure (10: 60: 30),
0,2 Gt einer der Verbindungen A i, A l, A m und A o,
0,15 Gt Triethylenglykol-dimethacrylat und
0,035 Gt des in Beispiel 1 angegebenen blauen Azofarbstoffs in

FIG9/100

30 Gt Butanon und
0,5 Gt Ethylalkohol
werden nacheinander auf eine 25µm starke Polyethylenterephthalatfolie so aufgeschleudert, daß eine 25µm (30 g/m²) dicke Schicht erhalten wird. Anschließend wird 2 Minuten bei 100°C im Trockenschrank nachgetrocknet.

Um die erhaltenen Schichten vor Verschmutzung durch Staub und Beschädigungen zu schützen, werden sie mit einer 20 - 25 µm starken Deckfolie, die an der Schicht weniger stark haftet als die Polyester-Trägerfolie, abgedeckt. Sie können so über einen längeren Zeitraum gelagert werden.

Die Kupferoberfläche einer mit 35µm starker Kupferfolie kaschierten Phenoplast-Schichtstoffplatte wird mechanisch mit Bimsmehl oder mit einer Bürstenmaschine gereinigt und nach intensivem Abspülen mit Wasser mit ölfreier Luft trockengeblasen.

Auf die vorgereinigten Cu-Platten wird der Trockenresist - nach Abziehen der Deckfolie - mit Hilfe eines mit beheizten Walzen bestückten Laminators bei 120°C und mit einer Geschwindigkeit von 1,5 m/min laminiert.

Alle vier Proben werden anschließend durch die Trägerfolie unter einem 13-stufigen Belichtungskeil mit Dichteinkrementen von 0,15 mit einer 5 kW-MH-Lampe 10, 20 und 40 Sekunden belichtet.

Die Keilstufe 0 entspricht der optischen Dichte 0,05 (Eigen-Absorption des Filmmaterials).

Nach Abziehen der Ttägerfolie werden die Platten einer Sprühentwicklung mit 0,8 %iger Sodalösung unterworfen. Die Entwicklungszeit beträgt ca. 60 Sekunden bei 23°C.

Um die Entwicklerresistenz zu prüfen - ein Test, um festzustellen, ob die Keilstufen voll vernetzt sindwurden die Proben nach einer Belichtung von 20 Sekunden der 3-fachen Entwicklungszeit - also 180 Sekundenunterworfen.

Die voll vernetzten Keilstufen der Trockenresistschichten, die sich nur durch den Photoinitiator unterscheiden, sind in folgender Tabelle zusammengefaßt:

| Verbindung | Vollstufen im Sprühgerät bei | | | Vollstufen bei |
|---|---|---|---|---|
| | 10 Sek. | 20 Sek. | 40 Sek. | 180 Sekunden |
| | Belichtung | | | Entwicklung |
| A i | 2 | 4 | 6 | 3 |
| l | 1 | 3 | 5 | 2 |
| m | 2 | 4 | 6 | 3 |
| o | 1 | 3 | 4 | 2 |

**Beispiel 3**

Je 0,4 g der Verbindungen A i, A l, A m und A o werden wie in Beispiel 2 beschrieben on Photopolymergemische eingearbeitet und die Lösungen auf 25 µm starke Polyethylenterephthalatfolien so aufgeschleudert, daß 25 µm (30 g/m²) dicke Schichten erhalten werden.

Die Schichten werden in gleicher Weise wie in Beispiel 2 auf die gereinigte Cu-Oberfläche von 10 x 15 cm großen Cu-Pertinax-Platten aufgebracht, durch die Trägerfolie belichtet und mit 0,8 %iger wäßriger Sodalösung entwickelt.

Es ergeben sich folgende vollvernetzte Keilstufen (zum Vergleich dahinter: Vollstufen bei Zugabe von je 0,6 g/ Lösung):

| Verbindung | Vollstufen bei 0,4 g Initiator Belichtungszeit, Sekunden | | | Vergleich: bei 0,6 g Initiator Belichtungszeit, Sekunden | | |
|---|---|---|---|---|---|---|
| | 10 | 20 | 40 | 10 | 20 | 40 |
| A i | 4 | 6 | 8 | 3 | 5 | 7 |
| l | 3 | 5 | 7 | 2 | 4 | 6 |
| m | 4 | 6 | 8 | 2 | 4 | 7 |
| o | 2 | 4 | 6 | 1 | 3 | 5 |

**Beispiel 4**

Eine Lösung von

1,0 Gt Trimethylolethantriacrylat,

1,4 Gt eines Terpolymerisats aus n-Hexylmethacrylat/ Methylmethacrylat/Methacrylsäure (50: 25: 25) mit einer Säurezahl von ca. 160,

0,02 Gt Sudanblau II und

0,05 Gt Verbindung A i in

6,0 Gt Butanon

wird auf eine gesäuberte Einstufen-Zinkätzplatte so aufgeschleudert und getrocknet, daß ein Schichtgewicht von ca. 10g/$m^2$ erhalten wird.

Anschließend wird das Kopiermaterial mit einem Überzug von 1 - 2µm Polyvinylalkohol versehen, getrocknet und 40 Sekunden unter einer Positiv-Vorlage mit einer MH-Lampe von 5 kW belichtet. Die Zinkplatte wird 45 Sekunden mit einem Entwickler aus

1,5 Gt Natriummetasilikat-Nonahydrat,

0,3 Gt Polyglykol 6000,

0,3 Gt Lävulinsäure,

0,3 Gt Strontiumhydroxid x 8 $H_2O$ und

97,6 Gt entsalztem Wasser

entwickelt.

Nach gründlicher Wasserspülung wird 5 Minuten mit 10 %iger Salpetersäure unter Zusatz von Flankenschutzmittel geätzt. Die gehärtete Photopolymerschicht wird mit Ethylenglykolmonobutylether entfernt. Die so entstandene Druckform eignet sich für den Qualitätsbuchdruck.

**Beispiel 5**

Eine Beschichtungslösung wie in Beispiel 4, jedoch mit 0,05 Gt der Verbindung A m anstelle der Verbindung A i wird auf eine 25 µm starke Polyethylenterephthalatfolie durch Gießen so aufgetragen, daß eine 20 µm (26 g/$m^2$) dicke Schich erhalten wird. Anschließend wird 2 Minuten bei 100°C im Trockenschrank nachgetrocknet.

Die getrocknete Schicht wird zusammen mit der Polyesterfolie mittels eines Laminators bei höchstem Andruck und einer Temperatur von 115°C mit einer Geschwindigkeit von 1 m/min auf Siebdruckgewebe VS-Monoprint P 77 der Firma Verseidag, Krefeld, auflaminiert.

Anschließend wird durch die Polyesterfolie 60 Sekunden mit einer 5 kW-MH-Lampe unter einer Positiv-Vorlage belichtet.

Nach Entfernen der Polyesterfolie werden mit dem in Beispiel 4 beschriebenen Entwickler innerhalb von 45 Sekunden im Schaukelbad die unvernetzten Bildbereiche entfernt. Nach gründlicher Wasserspülung und Trocknung ist die Siebdruckform gebrauchsfertig.

**Patentansprüche**

1. Photopolymerisierbares Gemisch, das als wesentliche Bestandteile

(a) ein polymeres Bindemittel,

(b) eine polymerisierbare Verbindung mit mindestens zwei endständigen ethylenisch ungesättigten Gruppen und mit einem Siedepunkt oberhalb 100°C und

(c) als Photoinitiator eine mehrkernige N-heterocyclische Verbindung

enthält, dadurch gekennzeichnet, daß die N-heterocyclische Verbindung der Formel A

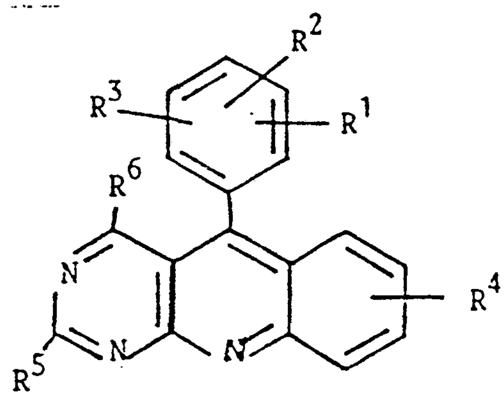

(A)

entspricht, worin

$R^1$, $R^2$ und $R^3$ gleich oder verschieden sind und jeweils ein Wasserstoff- oder Halogenatom, eine Alkyl- oder Alkoxygruppe mit jeweils 1 bis 6 Kohlenstoffatomen oder einen ankondensierten aromatischen Rest,

$R_4$ ein Wasserstoff- oder Halogenatom, eine Alkyl-, Alkoxy-, Halogenalkyl-, Alkylcarbonyl-, Alkoxycarbonylgruppe mit jeweils 1 bis 6 Kohlenstoffatomen in der Alkyl- oder Alkoxygruppe, eine Dialkylaminogruppe mit 2 bis 10 Kohlenstoffatomen oder einen ankondensierten aromatischen Rest und

$R^5$ und $R^6$ gleich oder verschieden sind und Hydroxy-, oder Alkoxygruppen mit 1 bis 6 Kohlenstoffatomen oder Dialkylaminogruppen mit 2 bis 10 Kohlenstoffatomen

bedeuten.

2. Photopolymerisierbares Gemisch nach Anspruch 1, dadurch gekennzeichnet, daß es eine Verbindung der Formel A enthält, worin $R^1$, $R^2$ und $R^3$ Wasserstoffatome sind.

3. Photopolymerisierbares Gemisch nach Anspruch 1, dadurch gekennzeichnet, daß es eine Verbindung der Formel A enthält, worin $R^6$ eine Hydroxy- oder Alkoxygruppe ist.

4. 10-Phenyl-1,3,9-triazaanthracene der allgemeinen Formel A

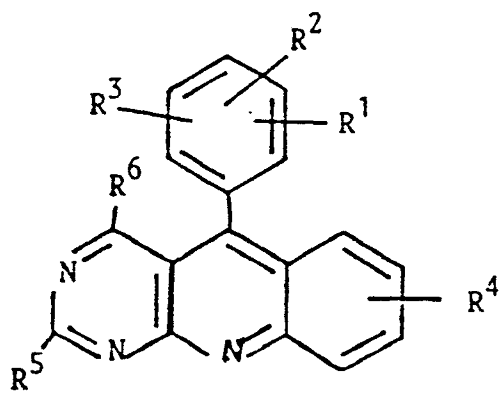

(A)

worin

$R^1$, $R^2$ und $R^3$ gleich oder verschieden sind und jeweils ein Wasserstoff- oder Halogenatom, eine Alkyl- oder Alkoxygruppe mit jeweils 1 bis 6 Kohlenstoffatomen oder einen ankondensierten aromatischen Rest,

$R_4$ ein Wasserstoff- oder Halogenatom, eine Alkyl-, Alkoxy-, Halogenalkyl-, Alkylcarbonyl-, Alkoxycarbonylgruppe mit jeweils 1 bis 6 Kohlenstoffatomen in der Alkyl- oder Alkoxygruppe, eine Dialkylaminogruppe mit 2 bis 10 Kohlenstoffatomen oder einen ankondensierten aromatischen Rest und

$R^5$ und $R^6$ gleich oder verschieden sind und Hydroxy-, oder Alkoxygruppen mit 1 bis 6 Kohlenstoffatomen oder Dialkylaminogruppen mit 2 bis 10 Kohlenstoffatomen

bedeuten.

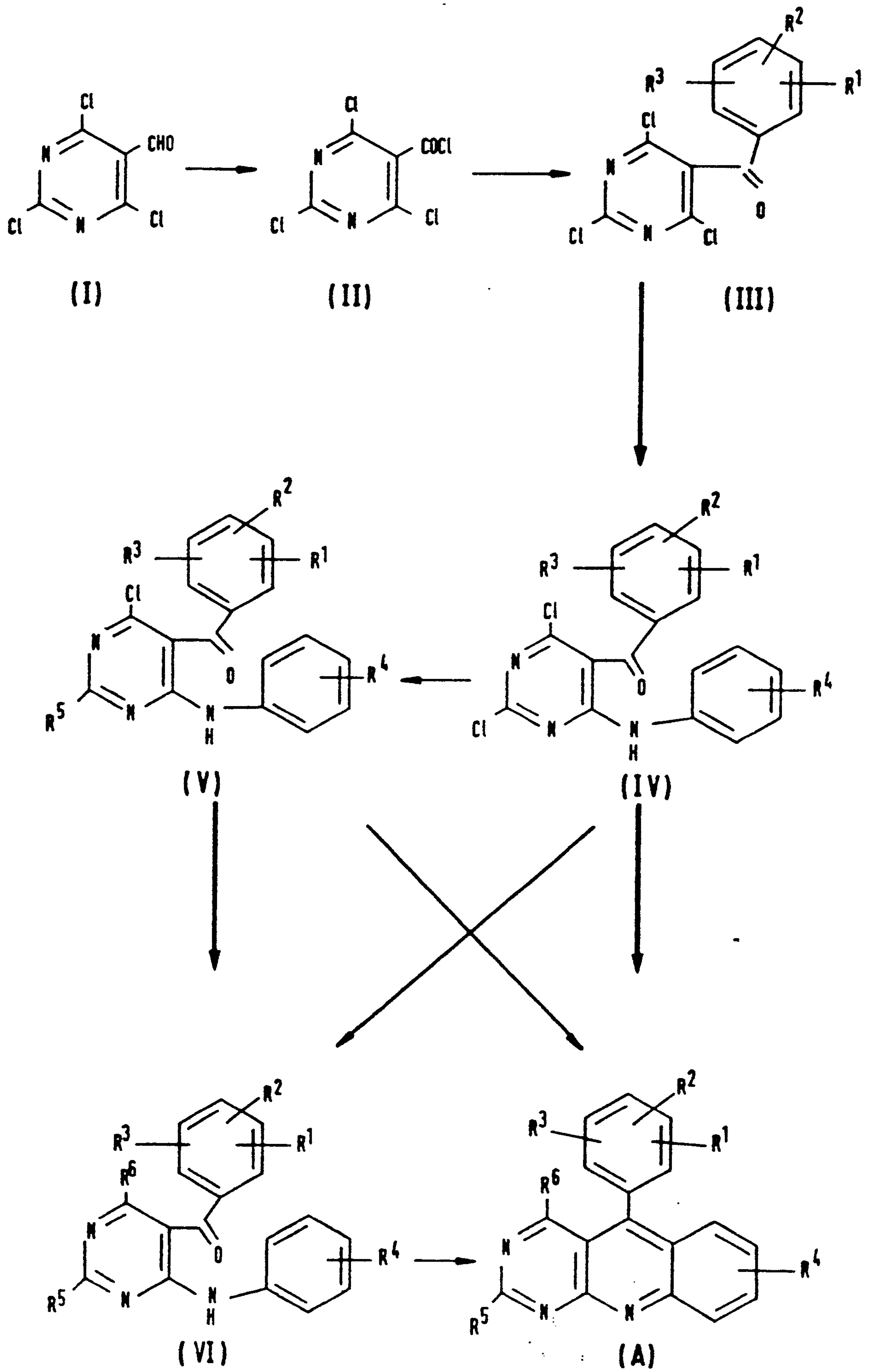

(I) (II) (III)

(V) (IV)

(VI) (A)

**Claims**

1. Photopolymerizable mixture which contains, as essential components,
a) a polymeric binder,
b) a polymerizable compound having at least two terminal ethylenically unsaturated groups and a boiling point of more than 100° C, and
c) a polynuclear N-heterocyclic compound as the photoinitiator,
characterized in that the N-heterocyclic compound corresponds to the general formula A

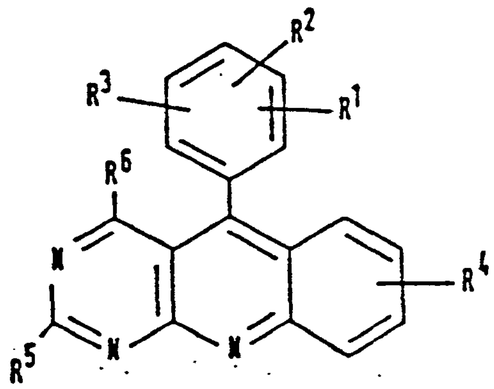

(A)

wherein
$R^1$, $R^2$ and $R^3$ are identical or different and each denote a hydrogen or halogen atom, an alkyl or alkoxy group each having from 1 to 6 carbon atoms, or a condensed aromatic radical,
$R^4$ is a hydrogen or halogen atom, an alkyl, alkoxy, halogenoalkyl, alkylcarbonyl, or alkoxycarbonyl group each having from 1 to 6 carbon atoms in the alkyl or alkoxy groups, respectively, a dialkylamino group having from 2 to 10 carbon atoms, or a condensed aromatic radical, and
$R^5$ and $R^6$ are identical or different and denote hydroxy or alkoxy groups each having from 1 to 6 carbon atoms or dialkylamino groups having from 2 to 10 carbon atoms.

2. Photopolymerizable mixture as claimed in Claim 1, characterized in that it contains a compound of the general formula A, wherein $R^1$, $R^2$ and $R^3$ are hydrogen atoms.

3. Photopolymerizable mixture as claimed in Claim 1, characterized in that it contains a compound of the general formula A, wherein $R^6$ is a hydroxy group or an alkoxy group.

4. 10-phenyl-1,3,9-triazaanthracenes of the general formula A

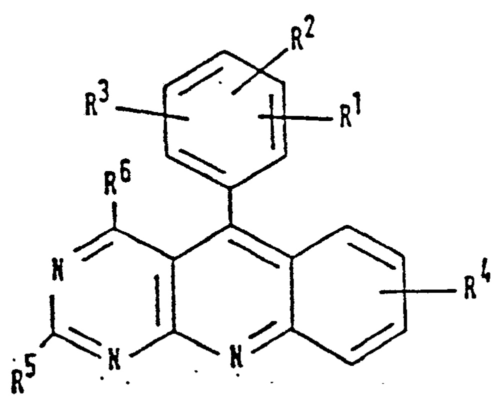

(A)

wherein
$R^1$, $R^2$ and $R^3$ are identical or different and each denote a hydrogen or halogen atom, an alkyl or alkoxy group each having from 1 to 6 carbon atoms, or a condensed aromatic radical,
$R^4$ is a hydrogen or halogen atom, an alkyl, alkoxy, halogenoalkyl, alkylcarbonyl, or alkoxycarbonyl group each having from 1 to 6 carbon atoms in the alkyl or alkoxy groups, respectively, a dialkylamino group, having from 2 to 10 carbon atoms, or a condensed aromatic radical, and
$R^5$ and $R^6$ are identical or different and denote hydroxy or alkoxy groups each having from 1 to 6 carbon atoms or dialkylamino groups having from 2 to 10 carbon atoms.

**Revendications**

1. Mélange photopolymerisable, qui contient en tant que constituants essentiels
(a) un liant polymère,
(b) un composé polymérisable possédant au moins deux groupes terminaux à insaturation éthylénique et ayant un point d'ébullition supérieur à 100°C et
(c) un composé N-hetérocyclique polynucléaire en tant que photo-amorceur, caractérisé par le fait que le composé N-hétérocyclique correspond à la formule A

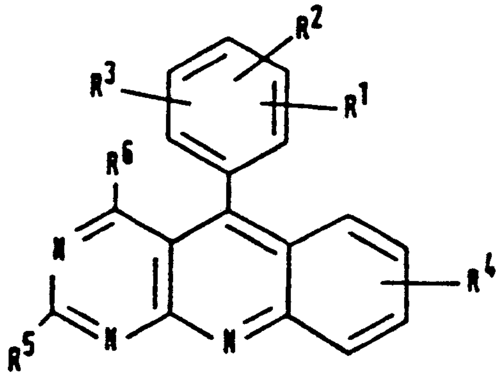

(A)

dans laquelle
$R^1$, $R^2$ et $R^3$ sont identiques ou différents et représentent chacun un atome d'hydrogène ou d'halogène, un groupe alkyle ou alcoxy ayant chaque fois de 1 à 6 atomes de carbone, ou un radical aromatique condense;
$R^4$ représente un atome d'hydrogène ou d'halogène, un groupe alkyle, alcoxy, halogéno-alkyle, alkylcarbonyle, alcoxycarbonyle contenant chaque fois de 1 à 6 atomes de carbone dans le radical alkyle ou alcoxy, un groupe dialkylamino ayant de 2 à 10 atomes de carbone, ou un radical aromatique condensé; et
$R^5$ et $R^6$ sont identiques ou différents et représentent des groupes hydroxy ou alcoxy ayant de 1 à 6 atomes de carbone ou des groupes dialkylamino contenant de 2 à 10 atomes de carbone.

2. Mélange photopolymérisable selon la revendication 1, caractérisé en ce qu'il contient un composé de formule A dans lequel R , $R^2$ et $R^3$ sont des atomes d'hydrogène.

3. Mélange photopolymérisable selon la revendication 1, caractérisé en ce qu'il contient un composé de formule A dans lequel $R^6$ est un groupe hydroxy ou alcoxy.

4. 10-phényl-1,3,9-triaza-anthracènes de formule générale A

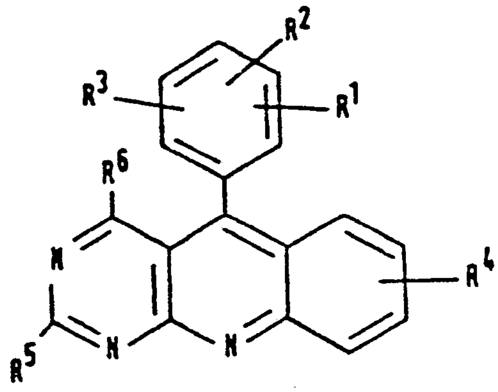

(A)

dans laquelle
$R^1$, $R^2$ et $R^3$ sont identiques ou différents et représentent chacun un atome d'hydrogène ou d'halogène, un groupe alkyle ou alcoxy ayant chaque fois de 1 à 6 atomes de carbone, ou un radical aromatique condense;
$R^4$ represente un atome d'hydrogène ou d'halogène, un groupe alkyle, alcoxy, halogéno-alkyle, alkylcarbonyle, alcoxycarbonyle contenant chaque fois de 1 à 6 atomes de carbone dans le radical alkyle ou alcoxy, un groupe dialkylamino ayant de 2 à 10 atomes de carbone, ou un radical aromatique condensé; et
$R^5$ et $R^6$ sont identiques ou différents et représentent des groupes hydroxy ou alcoxy ayant de 1 à 6 atomes de carbone ou des groupes dialkylamino contenant de 2 à 10 atomes de carbone.